(19)

**Europäisches Patentamt**
**European Patent Office**
**Office européen des brevets**

(11) **EP 1 872 422 B1**

(12) **EUROPEAN PATENT SPECIFICATION**

(45) Date of publication and mention of the grant of the patent:
**20.06.2012 Bulletin 2012/25**

(51) Int Cl.:
*H01M 4/02* (2006.01)   *H01M 4/04* (2006.01)
*A61N 1/05* (2006.01)

(21) Application number: **05722304.2**

(22) Date of filing: **31.03.2005**

(86) International application number:
**PCT/SE2005/000480**

(87) International publication number:
**WO 2006/104432 (05.10.2006 Gazette 2006/40)**

(54) **POROUS NIOBIUM OXIDE AS ELECTRODE MATERIAL AND MANUFACTURING PROCESS**

PORÖSES NIOBOXID ALS ELEKTRODENMATERIAL UND HERSTELLUNGSPROZESS

OXYDE DE NIOBIUM POREUX EN TANT QUE MATERIAU POUR ELECTRODE ET PROCEDE DE FABRICATION

(84) Designated Contracting States:
**AT BE BG CH CY CZ DE DK EE ES FI FR GB GR HU IE IS IT LI LT LU MC NL PL PT RO SE SI SK TR**

(43) Date of publication of application:
**02.01.2008 Bulletin 2008/01**

(73) Proprietor: **St. Jude Medical AB**
**175 84 Järfälla (SE)**

(72) Inventor: **NORLIN, Anna**
**S-113 52 STOCKHOLM (SE)**

(56) References cited:
**US-A- 2004 256 146**

• **FISCHER V. ET AL.: 'Niobium as New Material for Elecolyte Capacitors with Nanoscale Dielectric Oxide Layers.' PROCEEDINGS OF THE 7TH INTERNATIONAL CONFERENCE vol. 3, 01 June 2003, pages 1134 - 1137, XP010649393**
• **YEROKHIN A. ET AL.: 'Plasma electrolysis for surface engineering.' SURFACE AND COATINGS TECHNOLOGY vol. 122, 1999, pages 73 - 93, XP002992080**
• **DATABASE PRIORART IPCOM000021843D, 11 February 2004 'Fully Implantable Miniature Stimulator for Stimulation of Respiration', XP013014320 & IP.COM ELECTRONIC PUBLICATION 11 February 2001, pages 1 - 14**
• **DATABASE PRIORART IPCOM000042621D, 01 June 1984 'Plasma Technique for Thin Oxide Film Growth.', XP002992081 & IP.COM ELECTRONIC PUBLICATION no. 13-17, 04 February 2005, pages 60 - 62**
• **LEONI R. ET AL.: 'Sub-micron niobioum electrodes for dielectrophoresis applications.' MICROELECTRONIC ENGINEERING vol. 30, 1996, pages 555 - 558, XP000588046**

**Description**

TECHNICAL FIELD OF THE INVENTION

The present application concerns an electrode having as surface a new material and a process for producing this new material. The electrode of the invention is suitable for use in the human or animal body. The new material gives the electrode excellent electrochemical properties.

BACKGROUND OF THE INVENTION

Individuals suffering from certain heart conditions (e. g. arrhythmia) can restore normal heart rhythm and improve life quality by having a pacemaker implanted. Pacemakers generate electrical impulses that artificially stimulate the heart tissue. The impulses are transferred to the heart by an electrode, often located inside the heart. On the electrode/ tissue interface the electrical stimulation current is converted to an ionic current which is able to act in the body. There are three electrochemical mechanisms by which the current can be transferred over the interface: i) non-faradaic charging/ discharging of the electrochemical double layer, ii) reversible and iii) irreversible faradaic reactions. The first two mechanisms do not generate by-products and are preferred rather than irreversible reactions, which generate by-products that can be harmful to the body.

To reduce battery drain and increase the lifetime of the pacemaker, the amount of energy delivered by each stimulation pulse should be small. One way to achieve low energy stimulation is to increase the impedance of the electrode by coating it with a material of high dielectric constant. These materials have high ohmic resistance and will inhibit electron transfer through electrochemical reactions, but can transfer stimulation pulses by a capacitive current. In this way, irreversible electrochemcial reactions leading to charge loss are avoided, which is also beneficial as there are no net-reactions.

This type of materials has been used as electrode materials in capacitors due to their charge storage properties. For example V. Fischer, H. Stormer, D. Gerthsen, M. Stenzel, H. Zilleen, E. Ivers-Tiffee describe the dielectric properties of a niobium oxide layer in "Niobium as new material for electrolyte capacitors with nanoscale dielectric oxide layers", Proceedings of the IEEE International Conference on Properties and Applications of Dielectric Materials, v 3 (2003), p 1134-1137. Several attempts have been made to make such electrodes work in reality for pacemaker applications, but without success.

The surface structure is important to implant materials in general and to stimulation electrodes in particular. It well known that surfaces exhibiting specific topography assists the in-growth of tissue and reduces the risk of inflammation. For pacemaker applications, it is also critical that the electrode is capable of delivering sufficient stimulation to ensure activation of the cardiac tissue. Traditionally, the charge delivery area has been increased in order to increase the surface capacitance. However, only a certain enlargement of the electrode area has proven to be useful during the high frequency stimulation processes. This implies certain limitations in traditional electrode design.

So called valve metals, i.e. Ti, Ta, W, Zr, Al, Hf, Nb, form a porous structure when subjected to high anodic potential (over dielectric break-down) pulses in an electrolyte. This process is called plasma electrolytic oxidation, anodic sparc oxidation or micro-arc oxidation (A. L. Yerokhin, X. Nie, A. Leyland, A. Matthews, S. J. Dowey, "Plasma electrolysis for surface engineering" Surface and Coatings Technology, v 122, n 2-3, 15 Dec. 1999, p 73-93) and has been used to produce corrosion and wear resistant surfaces. Recently, it has gained interest within the biomaterial industry due to its bioactive surface structures. The porous oxide formed on Ti exhibits good biocompatibility.

Pt, Ti and TiN coated electrodes are often used in pacemakers. For porous electrodes, only a fraction of the pores is accessible for the electrochemical processes at sufficiently high sweep rates. Owing to the effect of the distributed resistance inside the pores (IR drop), the available capacitance will diminish with increasing sweep rate. This means that, when the sweep rate is increased, the current in the cyclic volatmmetry eventually will change character from a near capacitive response to a near resistive response. Rough TiN coated electrodes show charge transfer limitations due to the IR drop. TiN is also very oxidation prone.

$Nb_2O_5$ is biocompatible and used as implant materials in a variety of applications. $Nb_2O$ is formed naturally on the metal in air. It can be grown by numerous methods including thermal oxidation, passivation in acid and by anodic oxidation. The anodically formed $Nb_2O_5$ -oxide on Nb is an oxygen deficient, highly doped n-type semiconductor with rectifying properties (the current flow in the cathodic direction but not in the anodic) an a bandgap of 3.4 eV.

The object of the invention is to overcome the limitations of presently used implantable electrodes, such as TiN coated electrodes. A further object is to obtain an implantable electrode having a high surface area, a high impedance, a high surface capacitance and good biocompatibility.

An electrode covered with a porous $Nb_2O_5$ -layer has surprisingly proved to be a very efficient electrode.

SUMMARY OF THE INVENTION

Thus, the invention concerns an electrode as defined in independent claim 1

This electrode may be produced according to the invention via the features of independent claim 9.

Surprisingly the porous oxide layer obtained on niobium is homogenous and does not show any cracks in spite of the severe treatment. An extensive high potential treatment with anodic pulses has led to flawed oxide layers on other metals (see for example A. Norlin, J. Pan, C. Leygraf, "Investigation of Electrochemical Behavior of Stimulation/Sensing electrode materials - I. Pt, Ti, and TiN-Coated Electrodes" submitted to Journal of Electrochemical Society (2004) and A. Norlin, J. Pan, C. Leygraf, "Investigation of Electrochemical Behavior of Stimulation/Sensing electrode materials - II. Conductive Oxide Coated Electrodes" submitted to Journal of Electrochemical Society (2004)).

The niobium oxide layer obtained by this process is new and has not been described earlier. It shows unexpected high performance when used as electrode layer, especially on a stimulation electrode.

BRIEF DESCRIPTION OF THE DRAWINGS

Figure 1 shows a SEM picture of $Nb_2O_5$ produced by plasma electrolytic oxidation by 125 pulses of 700 V and 10 ms duration.

Figure 2 shows diagrams over production of porous niobium oxide, a) potential response to applied pulses, b) current response to applied pulses,

Figure 3 shows SEM pictures of niobium oxide after a) 50 pulses, b) 75 pulses, c) 100 pulses and d) 125 pulses,

Figure 4 shows a SEM-picture of a cross-section of oxide in back-scatter mode,

Figure 5 shows Bode plots obtained from impedance spectroscopy of porous and smooth niobium in PBS,

Figure 6 shows Bode plots obtained from impedance spectroscopy of porous niobium in PBS,

Figure 7 shows an equivalent circuit used to model the porous $Nb_2O_5$ electrode and schematic illustration of two-layer oxide on niobium,

Figure 8 shows Bode plots of porous $Nb_2O_5$ during anodic polarization,

Figure 9 shows Bode plots of porous $Nb_2O_5$ during cathodic polarization,

Figure 10 is a diagram showing the change in $R_{bl}$ during cathodic polarization

Figure 11 a) is a cyclic voltammogram of porous $Nb_2O_5$ in PBS at 1, 5, 10, 15 and 20 V/s, and in b) capacitance is plotted vs potential for 1, 5, 10, 15 and 20 V/s,

Figure 12 a) is a diagram showing potential and b) a diagram showing current responses following applied pacemaker pulses of various magnitude,

Figure 13 a) is a diagram showing pulse impedance for various pulse potentials and b) a diagram showing delivered charge,

Figure 14 a and b are EIS spectra showing the sealing process of the pores during a) the first 30 days and b) 30-60 days,

Figure 15 a, b are cyclic voltammograms of porous niobium oxide obtained at 50 mV/s in PBS. a) shows change in current response with number of cycles, b) shows zoom of oxidation peak,

Figure 16 a-f are SEM pictures showing the niobium oxide obtained after 50, 75, 100, 125, 150 and 200 pulses, respectively.

DETAILED DESCRIPTION OF THE INVENTION

The invention concerns an implantable electrode having an electrically conductive core covered by a stable biocompatible oxide barrier layer, said core comprising niobium and said oxide comprising porous niobium oxide. The oxide layer comprises suitably an inner compact oxide and an outer porous oxide. The porous oxide may have a pore size of 1 to 20 $\mu$m, preferably 2 to 15 $\mu$m, especially 3 to 10 $\mu$m. The thickness of the barrier oxide layer is suitably 0.5 to 15 $\mu$m, preferably 1 to 10 $\mu$m and especially 2 to 7 $\mu$m. The core comprises niobium. It may be an alloy containing niobium or an essentially pure niobium metal. It is also possible to have a core comprising an inner core made of another metal or metal alloy and covered by a layer of niobium or niobium alloy. Preferably at least the outer layer of the core is made of niobium, most preferably essentially pure niobium.

The invention combines the advantages of the homogenous porous structure of plasma electrolytic oxidation formed $Nb_2O_5$ with its good electrical properties, such as capacitive stimulation properties.

According to the invention the present electrode is produced by connecting a core of metal or metal alloy containing niobium as anode in an electrolyte and subjecting it to high potential anodic pulses. Suitable electrolytes are a phosphate buffered saline solution and a solution of calcium acetate and calcium glycerophosphate. The pulse magnitude is suitably about 100 to 2000 V, preferably 200 to 1000 V and especially 500 to 1000 V. The pulse duration is suitably about 1 to 20 ms, preferably 2 to 15 ms, especially about 7 to 13 ms. The number of pulses should be at least 40, preferably at least 50, and at most 700, preferably at most 500, especially at most 300. Most preferably the number of pulses is in the interval from 100 to 200.

Also implantable electrodes comprising an electrically conductive core covered by a stable biocompatible oxide barrier layer, where said core comprises one of the valve metals Ti, Ta, W, Zr, Al, Hf and said oxide comprises a porous oxide of this metal produced by high anodic pulses show the same advantages as the electrode having a porous niobium oxide surface.

In the following examples the invention is further illustrated.

EXAMPLES

In the following examples the performance as a biocompatible electrode with capacitive stimulation properties is investigated. Electrochemical impedance spectroscopy (EIS) is used to investigate the electrode/electrolyte interface and its changes during anodic and cathodic polarization. Cyclic voltammetry (CV) is performed at different sweep rates in the potential range from -2 V to 2 V vs. sat. Ag/AgCl, to account for processes occurring during the pacemaker pulse. Also the electrochemical response of the electrode when subjected to pacemaker pulses is evaluated. All electrochemical measurements are performed in a phosphate-buffered solution (PBS), with $Na^+$, $K^+$, and $Cl^-$ contents similar to those of blood. The surface microstructure is characterized by scanning electron microscopy (SEM) and atomic force microscopy (AFM). The surface composition is analyzed by X-ray photoelectron spectroscopy (XPS) and electron dispersive spectroscopy (EDS).

**Experimental**

The nano-porous oxide was investigated by EIS, CV, SEM/EDS, AFM and XPS.

Electrode materials

*Nb* - A 99.99% piece of 1 mm thickness (Alfa Aesa) was polished to surface smoothness of 4000 grit and cleaned 5 minutes by a mixture of ethanol, acetone and distilled water (40:40:20) in an ultrasonic bath.

Electrolyte and electrochemical cell

All measurements were performed at room temperature. The electrolyte used was a phosphate-buffered-saline (PBS) solution, adjusted to pH 7.4 with 1 M NaOH. The solution has a similar ion strength as blood, $[Na^+]$ = 0.17 M, $[K^+]$ = 0.01 M and $[Cl^-]$ =0.15 M. The cell was a standard three-electrode flat cell (EG&G PARC Flat Cell) with a saturated Ag/AgCl electrode as reference electrode and a Pt mesh as counter electrode. The cell was equipped with a stirrer, adjusted at 750 rpm to remove bubbles on the working electrode during CV-measurements.

Anodic oxidation

Polished and cleaned niobium foil was anodically oxidized with 45 - 125 pulses. A device (UHM0013) built by St Jude Medical AB, Sweden, was used to generate and deliver the high potential pulses. Each pulse had a magnitude of 700 V and 10±2 ms duration. The integrated energy of each pulse was 40 J. Four pulses were applied in series, followed by a two minutes rest period before another pulse series was applied. The voltage and current responses of the materials when exposed to the pulses were recorded by a digital oscilloscope (Tektronix TDS 420A).

The UHM0013-device supplies capacitively delivered pulses, i.e., the pulse starts with the maximum voltage and decays with time. The pulses are not of constant potential, which complicates the analysis of the electrochemical processes occurring during pulsing. The potential response of the materials was directly measured between the working electrode and the Ag/AgCl (sat. KCl) reference electrode. To minimize the potential drop due to the electrolyte resistance, the reference electrode was inserted in a Lugging capillary placed close to the material surface. The current response was measured indirectly by recording the potential over a resistor (15 Ω) connected in series with the test cell. The current was then calculated using ohms law.

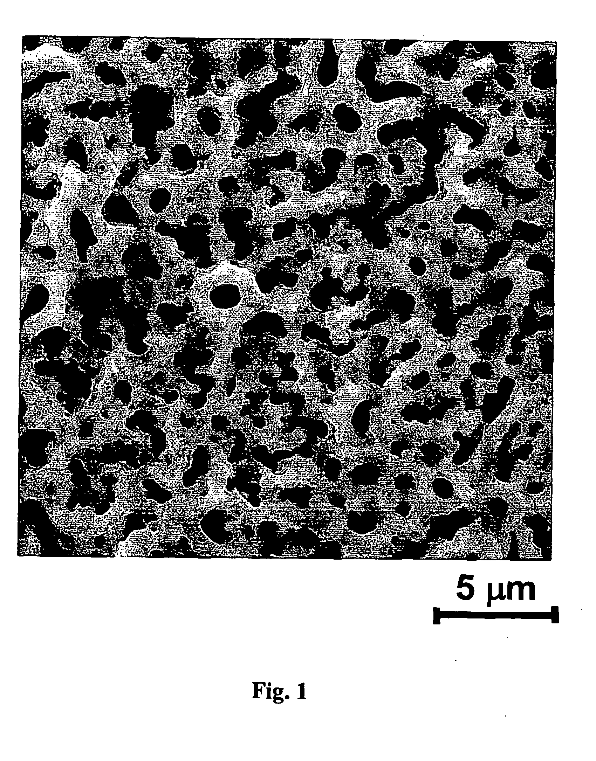

The niobium substrate anodically oxidized by 125 pulses of 700 V and 10 ms duration led to the highly porous $Nb_2O_5$ shown in the SEM picture in Figure 1.

Instruments and measurements

*Electrochemical impedance spectroscopy (EIS)* - EIS-measurements of the materials in the solution were performed to characterize the electrode/electrolyte interfaces. EIS-spectra were also obtained for the materials after simulated ageing. All EIS-spectra were collected using an electrochemical interface (Solartron 1287) and a frequency response analyser (Solartron 1250), controlled by a computer with ZPlot software (Scribner Associates, Inc.). The measurements were performed at the open circuit potential (OCP), over a frequency range from $1\times10^4$ to $5\times10^{-3}$ Hz. The perturbation amplitude was 10 mV. *Cyclic Voltammetry (CV)* - CV was used to study the electrochemical processes that may occur on the electrode surface. The measurements were performed by using either a Solartron 1287, controlled by a computer with CorrWare software (Scribner Associates, Inc.), or an EG&G 273A, controlled by PowerCV software. The CV cycling was performed between - 2 V and 2 V vs. Ag/AgCl (sat. KCl). To investigate the influence of the potential sweep rate, the cycling was performed using a range of sweep rates from 50 mV/s to 20 V/s (upper limit of the instrument/software systems).

*Pacemaker pulses* - Pacemaker pulses were applied between the sample (working electrode) and the counter electrode, using a pulse generator built by St Jude Medical AB, Sweden, which is able to deliver capacitively coupled stimulation and recharging pulses of adjustable potentials and frequencies. The voltage and current responses were recorded by using a digital oscilloscope (Tektronix TDS 420A). The potential response was directly measured between the working electrode and the reference electrode. To minimize the potential drop due to the electrolyte resistance, the reference electrode was inserted in a Lugging capillary and placed close to the sample surface. The responding transient current was measured by recording the potential over a resistor (0.1 Ω) connected in series with the test cell (counter electrode). The current was then calculated using ohms law.

*Surface analysis* - The surface structure of the electrode materials was examined by environmental SEM and AFM. The surface composition of selected samples was analyzed by XPS and EDS.

**Results and discussion**

Production and surface characterization

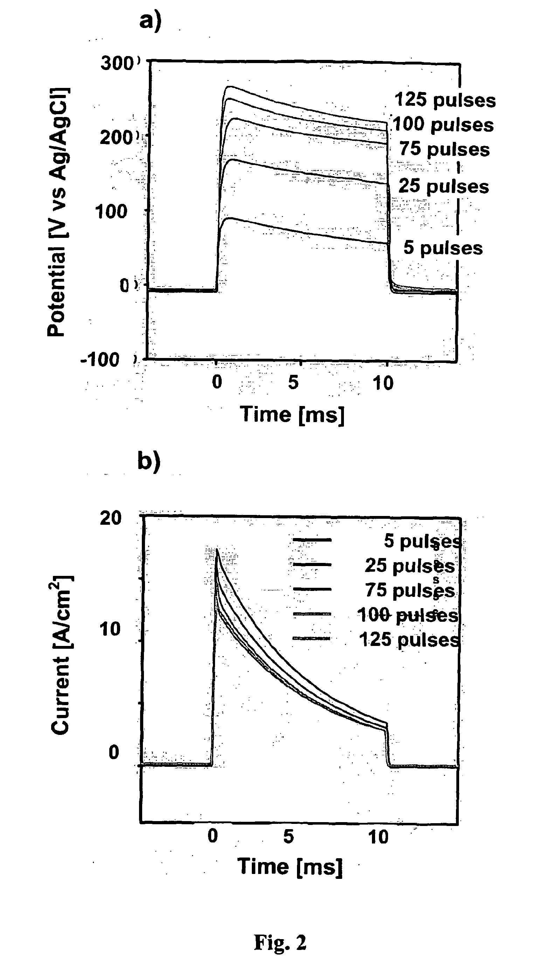

The polished niobium was subjected to high potential pulses of anodical polarity in PBS, according to the method described above. The potential and current responses of the material during pulsing were recorded (Fig. 2a-b). The surface potential increases with consecutive pulses as a consequence of the increasing thickness of the insulating oxide layer. In spite of the increasing surface potential, the current passing the interface decreases with consecutive pulses. Consequently, the charge passed in each pulse is decreased from 85 mC (pulse no 5) to 71 mC (pulse no 125). Calculation of oxide growth, based on 100% conversion factor and an oxide density of 4.36 g/cm$^3$ gives a 6 μm thick oxide layer formed.

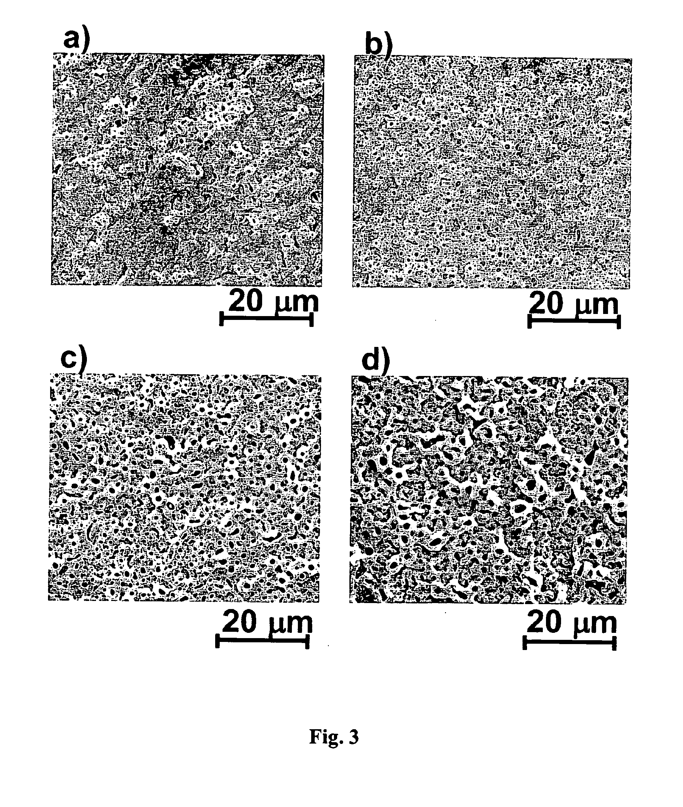

SEM pictures of surface morphology of niobium oxide after 50, 75, 100 and 125 pulses are shown in Figure 3a-d. After 50 pulses the oxide is covered to approximately 25 % by porous oxide. The surface is 100 % covered by homogeneous pores of about 1 μm diameter after 75 pulses. Scratch test showed that the oxide adheres well to the substrate and does not flake off close to the scratch. XPS- and XRD-examinations confirm that the oxide is $Nb_2O_5$ with nano-crystalline structure.

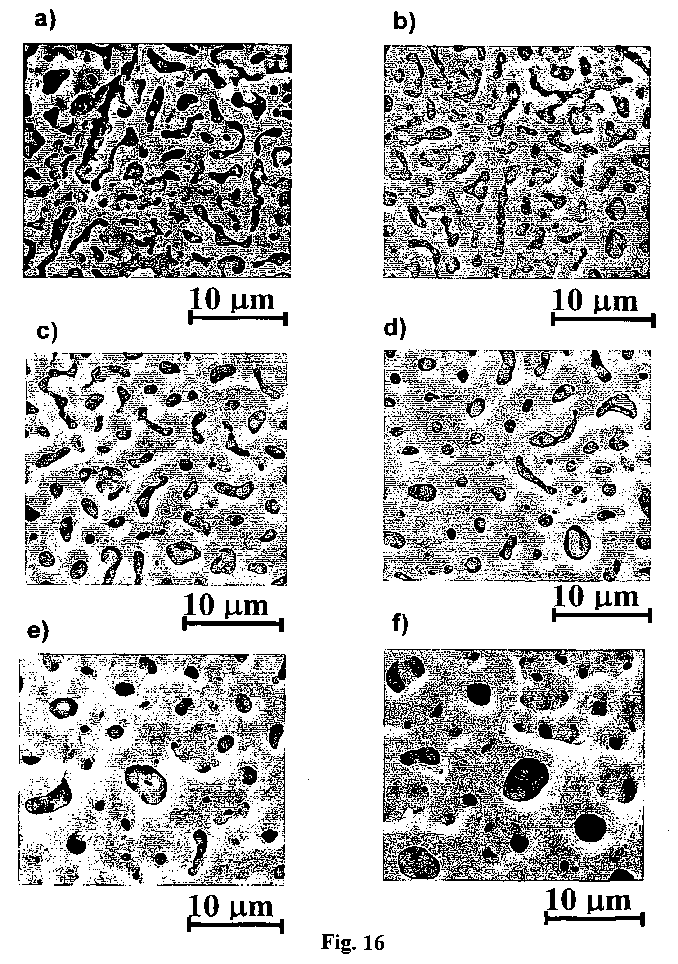

Figs. 16 a-f show another series of SEM-pictures taken after oxidation in PBS with 50, 75, 100, 125, 150 and 200 pulses, respectively, of 700 V. These SEM-pictures show clearly the changes in pore size and number of pores obtained when changing the number of pulses.

A niobium substrate was treated in an electrolyte consisting of a solution of calcium acetate and calcium glycerophosphate with anodic 700 V pulses. In this case P and Ca are incorporated in the oxide as shown I Table 1:

Table 1

| Pulses | O | P | Ca | Nb |
|---|---|---|---|---|
| 50 | 71.46 | 2.99 | 1.10 | 24.45 |
| 75 | 71.67 | 3.56 | 1.58 | 23.20 |
| 100 | 71.72 | 4.04 | 2.59 | 21.67 |
| 125 | 72.05 | 4.32 | 3.05 | 20.59 |
| 150 | 71.98 | 4.48 | 3.52 | 20.03 |
| 200 | 72.66* | 4.31* | 3.83* | 19.20* |

The values given are average values, except for the values given for 200 pulses (*) where the analysing apparatus broke down efter only one measurement.

The incorporation and concentration of elements such as P and Ca in the oxide may enhance the biocompatibility of the material.

Thus, by changing the number of pulses, the voltage, the electrolyte composition, etc, it is possible to control different oxide and pore parameters as well as thickness of the oxide layer.

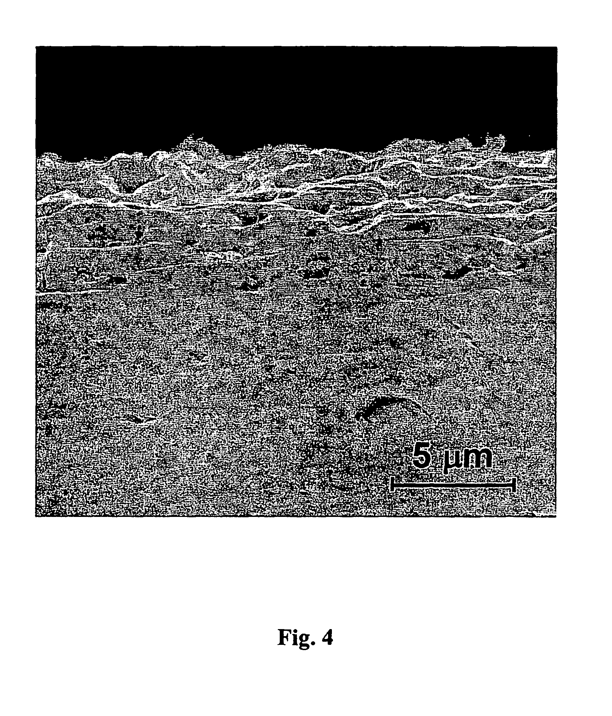

Cross-section of the samples show that the porous structure is approximately 2-3 $\mu$m thick (Fig 4). The compact barrier oxide is visible in the mixed SEM and backscatter picture where high atomic elements are shown as lighter gray shade than low atomic elements. Mapping of the chemical composition shows high oxygen content at about 5 $\mu$m down into the oxide, indicating a more dense oxygen layer.

EIS characterization of the porous oxide

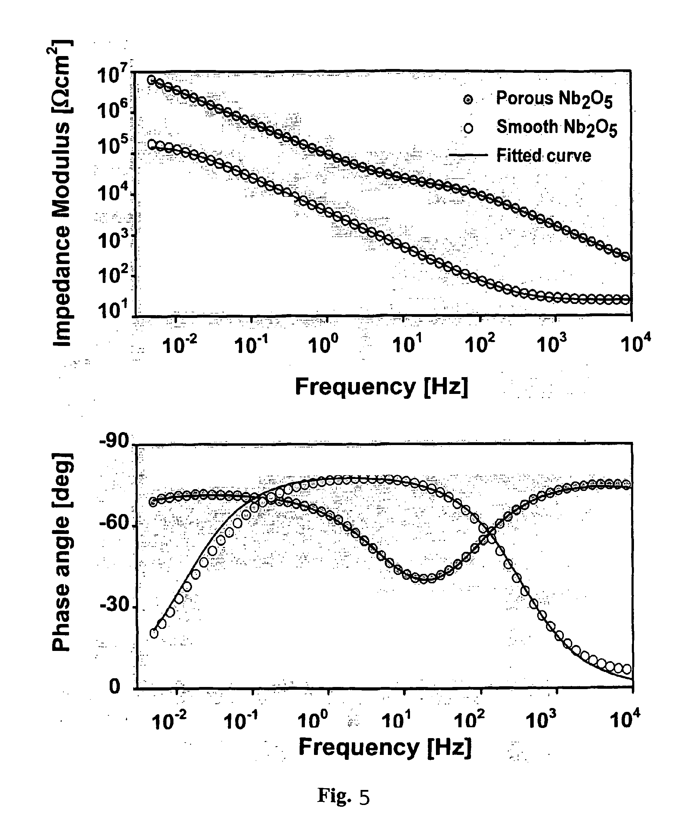

Investigation of the interface by EIS gives valuable information of the electrochemical behaviour of the material over a large frequency range. Only the EIS of niobium oxide after 125 pulses was investigated (100% covered surface). In Figure 5, the impedance response of smooth untreated niobium is compared to that of porous niobium oxide (125 pulses). The impedance spectra of the untreated smooth niobium electrode show characteristics of a high dielectric material, with high impedance at low frequencies implying a passive oxide on the electrode. The impedance modulus of the porous niobium is significantly higher as a result of the thicker oxide layer. The impedance spectrum of the porous niobium shows the behaviour of two time-constants, as evident by the two distinct peaks in phase angle. This confirms that the oxide film is a two-layer oxide, consisting of an inner compact barrier oxide and an outer porous oxide. This structure was confirmed by SEM in backscatter mode.

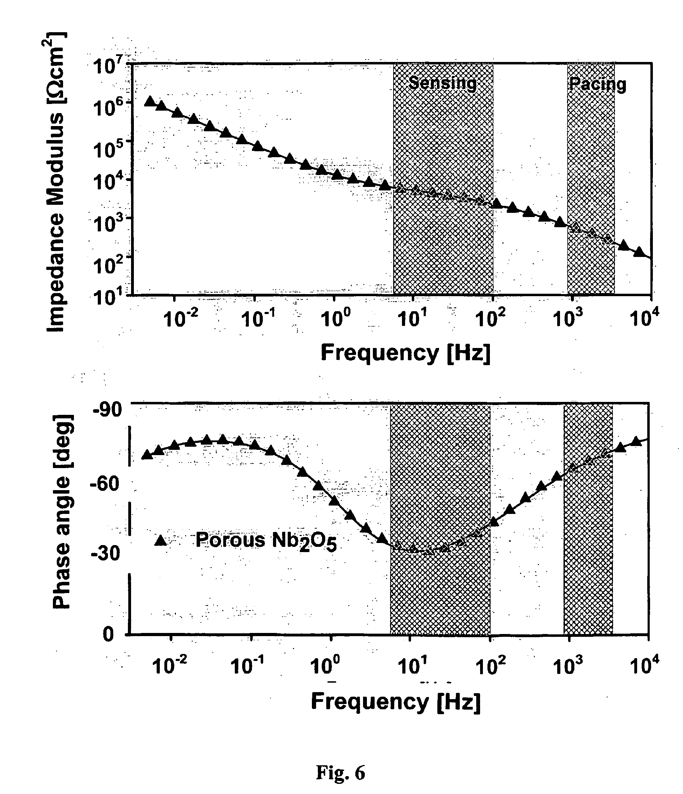

Stimulation and sensing processes take place at different frequencies. EIS-investigation can give useful information for designing pacemaker electrodes. Figure 6 shows impedance spectra in Bode format obtained for the porous $Nb_2O_5$, with the stimulation and sensing frequency ranges marked in grey.

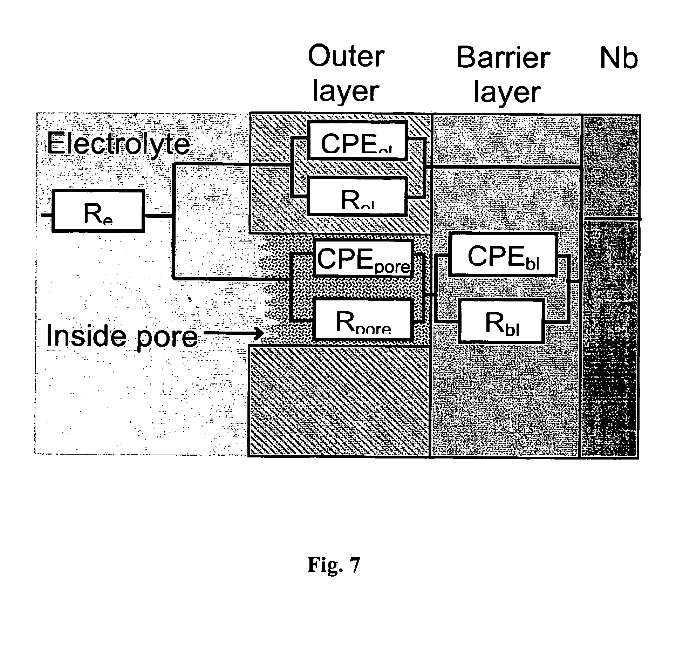

The choice of equivalent circuit is a two-layer model of an oxide film and has been applied in previous studies of oxide films on valve metals. In previous studies the impedance response from the pores was not separated from that of the outer oxide layer. However, to be able to identify the change in impedance response during anodic and cathodic polarization, and to be able to accurately analyze the impedance at intermediate frequencies, the more complex equivalent circuit in Figure 7 is used here. It is made up of six electric components where $R_e$ is the resistance of the electrolyte and $CPE_{ol}$, and $CPE_{bl}$ are the capacitances of the porous outer oxide layer and the barrier layer, respectively, and $R_{bl}$ is the resistances of the barrier layer. The resistance of the porous oxide layer is very high and could be omitted from the circuit without impairing the quality of the fitted spectra. These components satisfactorily describe the high and low frequency regions of the spectra, but the fit at intermediate frequencies is not very accurate. When a CPE in parallel with a resistor, $CPEp_{ore}$ and $R_{pore}$, was introduced into the circuit to account for the frequency dispersion within the pores a satisfactory fit was obtained over the total frequency range.

To account for non-ideal behaviour, the capacitance is represented by a constant phase element (CPE). The impedance of a CPE element is described by:

$$Z_{CPE} = \frac{1}{Q(i\omega)^{\eta}}$$

$$(1);$$

where i is the imaginary unit, $\omega$ the angular frequency, Q is a constant and $\eta$ is a mathematic expression ($0 \leq \eta \leq 1$). For an ideal capacitor, $\eta = 1$ and Q is the capacitance. The origin of CPE is due to geometric factors such as the roughness and porosity of the electrode as well as surface processes such as adsorption, surface reconstruction and diffusion.

By fitting the model to the experimental data, numeric values of the circuit components are obtained. In the high and low frequency region the impedance response is dominated by $CPE_{ol}$ and $CPE_{bl}$, respectively. The ranges of numeric values from the experiments are given in Table 2.

Table 2

| $CPE_{ol}$ | $\eta_{ol}$ | $CPE_{pore}$ | $\eta_{pore}$ | $CPE_{bl}$ | $\eta_{bl}$ | $R_{pore}$ | $R_{bl}$ |
|---|---|---|---|---|---|---|---|
| $\mu F$ | | $\mu F$ | | $\mu F$ | | $k\Omega.cm^2$ | $M\Omega.cm^2$ |
| 0.16-0.17 | 0.98-0.99 | 5-11 | 0.50-0.55 | 20-24 | 0.86-0.90 | 7-8 | 4-6 |

At OCP, the capacitance of the inner barrier oxide layer is 20-24 $\mu F/cm^2$. Assuming a dielectric constant of 42, the thickness of the barrier layer oxide is 1.6 - 1.9 nm, calculated by;

$$C = \varepsilon \varepsilon_0 \frac{A}{d} \qquad\qquad (2)$$

where $\varepsilon_0$ is the permittivity of vacuum ($8.85419 \times 10^{-14} F/cm$), A the area, and C the capacitance of the electrode. The polarization resistance of the barrier layer is high, around 5 $M\Omega cm^2$, indicating a high corrosion resistance, ie, a low rate of niobium release and oxide growth (J. Pan, D. Thierry, C. Leygraf, "Electrochemcial impedance spectroscopy study of the passive oxide film in titanium for implant applications", *Electrochim. Acta,* **41**, 1143 (1996)). The capacitance of the porous outer layer is low, 0.16-0.17 $\mu F/cm^2$ and the resistance of the electrolyte in the pore is in the range of 6-7 $k\Omega cm^2$. The best fit was obtained with $\eta_{pore}$ close to 0.5, which corresponds to a distributed RC transmission line model (network of distributed resistors and capacitors) which emphasizes the influence of diffusion processes inside the pores.

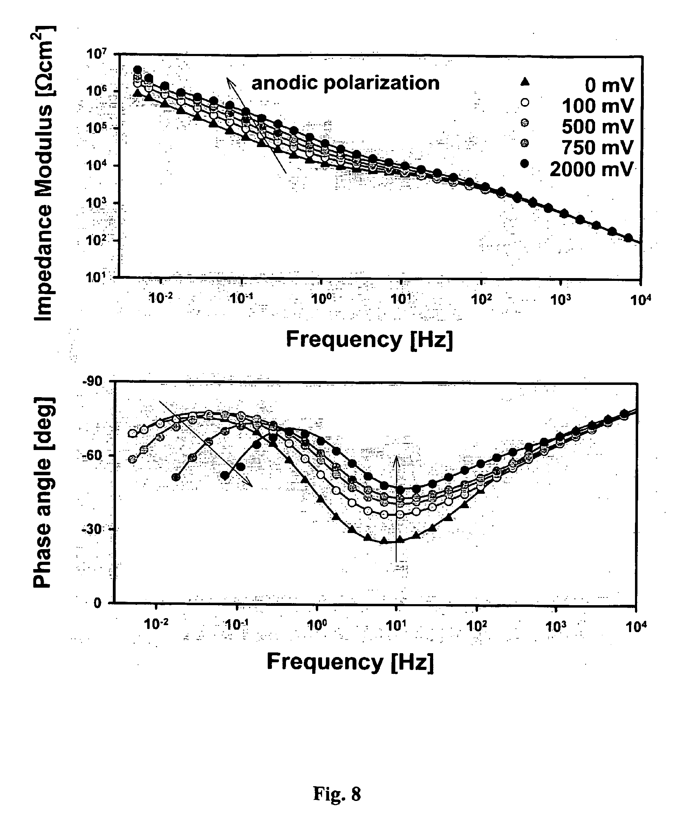

The impedance response during anodic polarization is shown in Bode format in Figure 8. At high frequencies the impedance modulus and phase angle is relatively unchanged for all magnitudes of polarizations. At low frequencies the impedance modulus increases, indicating some changing properties of the barrier layer.

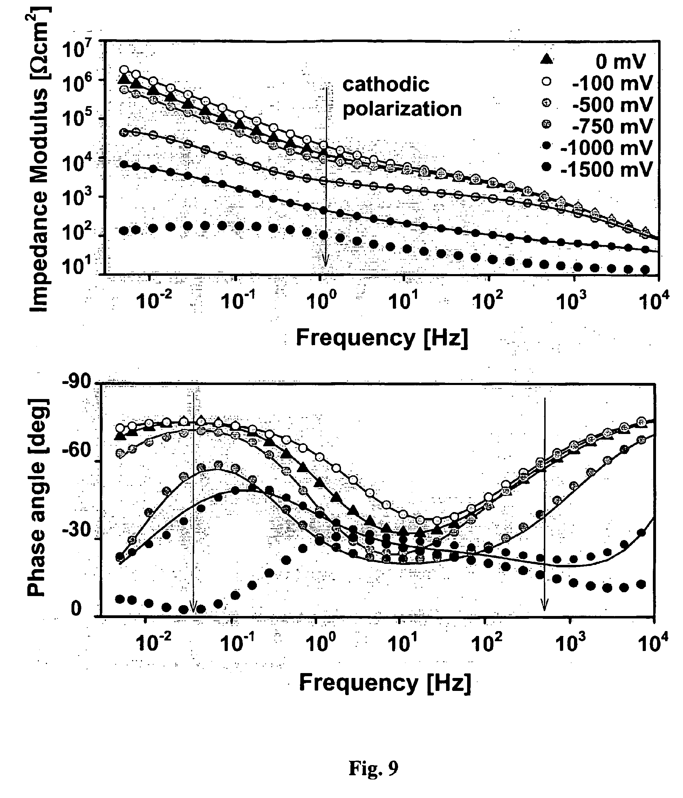

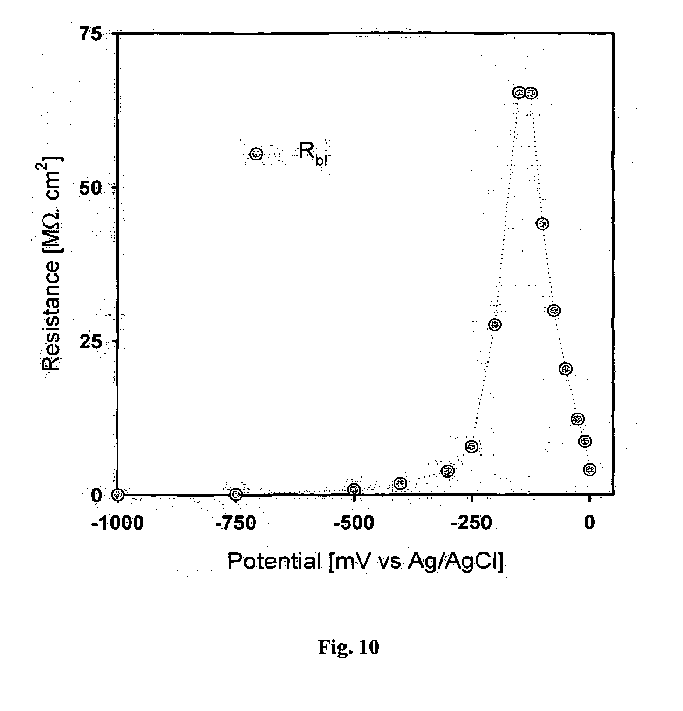

During cathodic polarization the impedance response and the phase angle decreases for both high and low frequencies, as shown in Figure 9. The resistivity of the barrier layer, $R_{bl}$, is at its maximum for -150 mV (Fig. 10), then decreases drastically for more cathodic potentials. The resistance within the pores initially increases when the potential become more cathodic until -250 mV, when it decreases again. The capacitance of the barrier layer increases with increasing cathodic polarization and at -750 mV it increases drastically, which might be a result of intercalation of hydrogen into the oxide.

Ageing in PBS solution

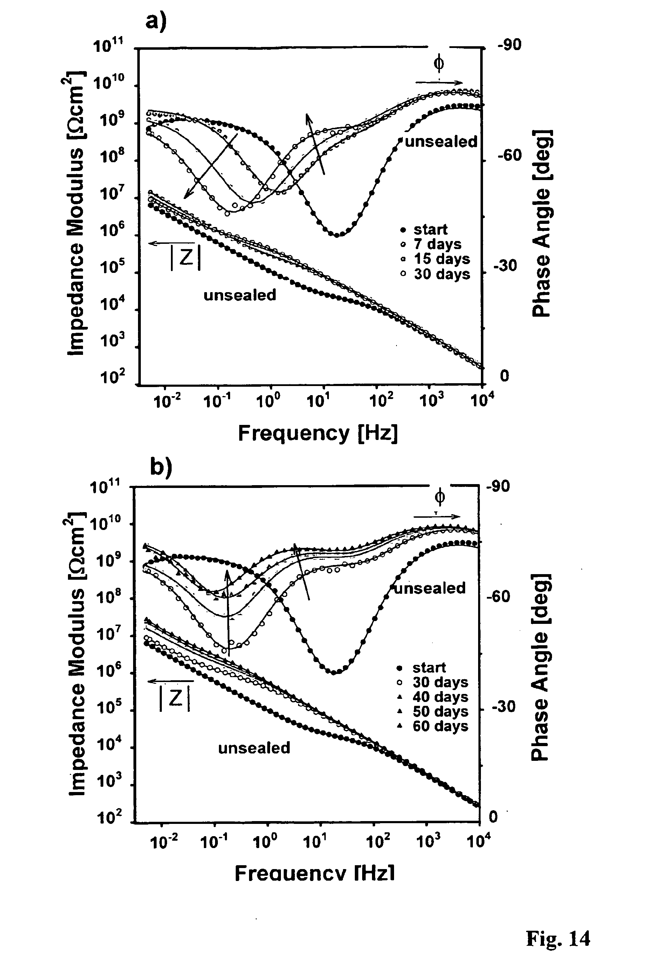

For the newly formed anodic oxide film, the pores are open and filled mainly with electrolyte, which is indicated by a low resistance of the precipitate, $R_{pore}$. The porous Nb oxide electrode was immersed in PBS for an extended period of time, and its change was monitored by EIS measurements. As shown in Fig. 14, a third peak in phase angle at medium frequencies developed with time. Such change in interfacial characteristics is commonly explained by precipitates filling the pores with time, a process known as pore sealing that occurs on anodic oxide films on valve metals. In this case, the porous part of the oxide film is gradually sealed, and the circuit elements representing the precipitates in the pores changes character.

The variation in the impedance response due to the aging of the porous Nb oxide in PBS provides detailed information about evolution of the oxide film.. After 30 days, the impedance modulus at low frequencies also rises (Fig. 14b) while $CPE_{bl}$ decreases, suggesting some thickening of the barrier oxide layer. $CPE_{ol}$ remain essentially unchanged through the exposure time. These results imply that the anodic Nb oxide layer is stable, and precipitates can fill the pores, leading to an increased corrosion resistance.

Cyclic voltammetry

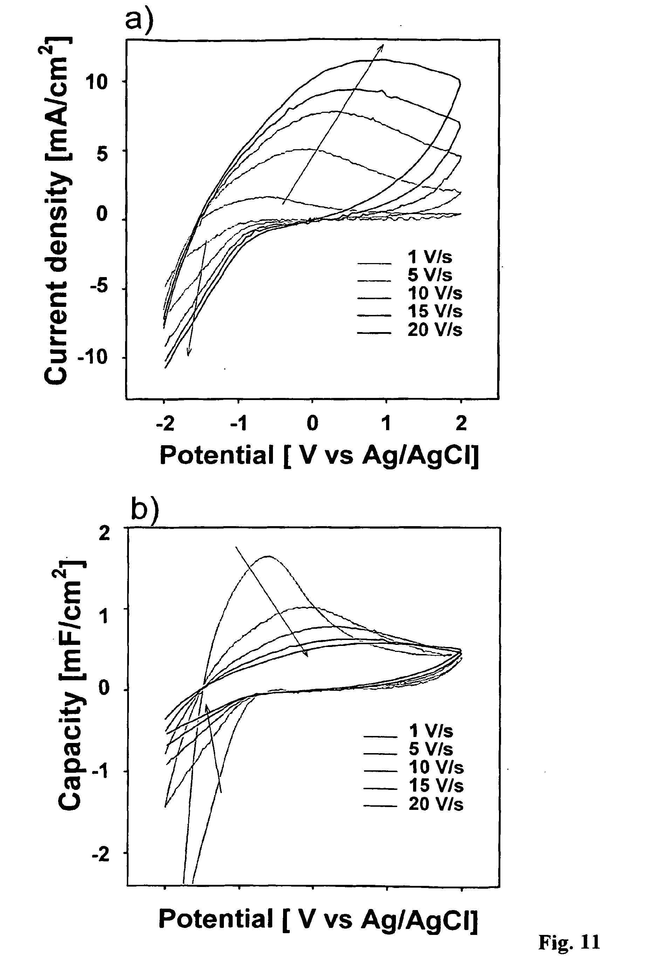

CV and capacitance curves (Figs. 11 a - b) of the porous $Nb_2O_5$ electrodes in the electrolyte show charging/discharging processes and the influence of the sweep rate. To reduce influences from changing film properties on consecutive scans, the film was pretreated by cycling 25 times at 20 V/s. For low sweep rates, very low currents pass the interface at anodic potentials and the current diminishes with increasing over-potential, indicating passive behaviour. For electrode potentials more cathodic than -0.7 V relatively large cathodic current flows, owing to a hydrogen evolution reaction. A small oxidation peak is visible around -1 V in the anodic scan direction, which may be attributed to oxidation of intercalated hydrogen. For increasing scan rates, the anodic oxidation peak becomes broader and more ill-defined and moves towards more anodic potentials for higher sweep rates. For sweep rates lower than 5 V the peak current is not linearly dependent on the sweep rate but for higher sweep rates a linearly dependence was found.

At high sweep-rates, the microstructure of the electrode plays an important role in the current response. When the sweep rate increases, the interface charging or discharging current will increase according to

$$i_{cap} = C_{interface} s \tag{3}$$

where the $C_{interface}$ is the combined double layer capacitance and the psuedo-capacitance of the surface bound redox-sites, and s is the sweep-rate. For porous electrodes, only a fraction of the pores is accessible for the electrochemical processes at sufficiently high sweep rates. Owing to the effect of the distributed resistance inside the pores (IR drop), the available capacitance will diminish with increasing sweep rate. This means that, when the sweep rate is increased, the current in the CV eventually will change character from a near capacitive response to a near resistive response.

Despite of its porous nature the $Nb_2O_5$ electrodes do not fully experience the effect of iR-drop limitations in charge/discharge transfer, for scan rates up to 20 V/s. Even at high scan rates, capacitive features are apparent in the CV. This is exemplified by the increasing current density with increasing scan rates, as well as the typical square like shape of the curves over certain potential ranges. Even though the capacitance diminishes with increasing scan rate, the decrease is not as pronounced as for porous electrodes relying only on charging/discharging of the electrochemical double layer for reversible charge transfer. This indicates that certain psuedo-capacitive redox reactions might contribute to the total charge transfer for $Nb_2O_5$ electrodes.

Thus, the porous $Nb_2O_5$ electrodes can utilise its capacitance more effectively at high discharge rates than rough TiN and porous carbon electrodes, which utilise about 5-10% of the capacitance measured by EIS.

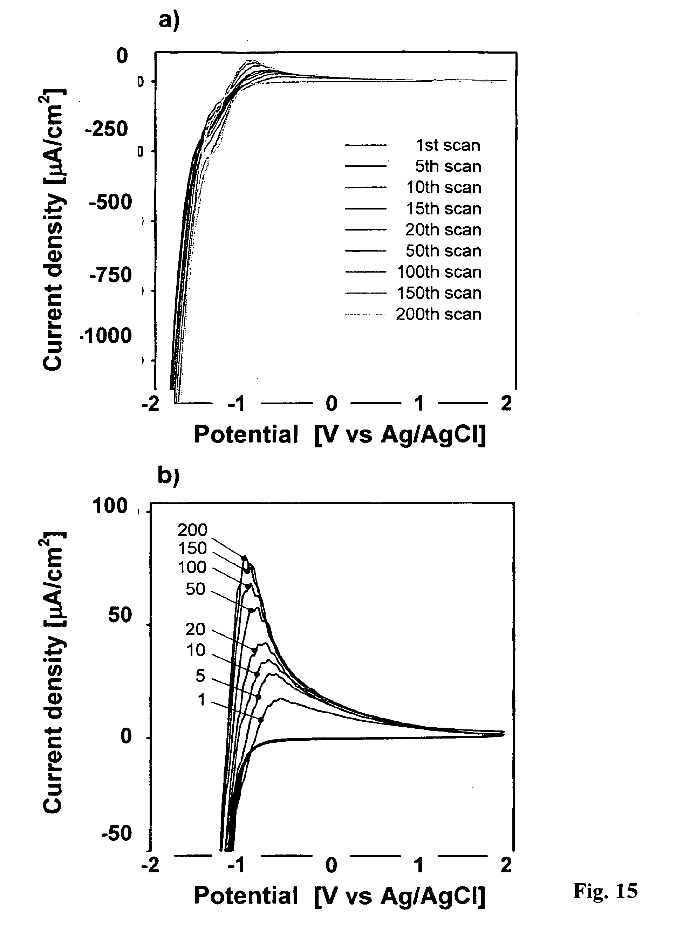

The CV of the porous Nb oxide electrode shows "rectifying" characteristics, Fig. 15a. At anodic potentials the current is very low and diminishes with increasing over-potential. At cathodic potentials below -1 V vs. Ag/AgCl, a distinct increase in current density appear, which can be explained by incorporation of H into the oxide and hydrogen evolution.

In the anodic sweep direction, an oxidation peak appears at about -0.6 V, which is due to oxidation of the H incorporated into the Nb oxide at the cathodic bias, a process known as hydrogen intercalation:

$$xH^+ + xe^- + NbO_{2.5} \longrightarrow H_x NbO_{2.5} \tag{5}$$

The current peak in the anodic sweep increases rapidly for the first 50 consecutive cycles (Fig. 15b), and then reaches a near constant level after 200 cycles. Meanwhile the oxidation peak is shifted to more cathodic potentials with increasing cycles indicating more easily oxidized species within the Nb oxide.

In the cathodic sweep direction, no corresponding reduction peak is observed, but the cathodic current increases significantly around -1 V, followed by a further pronounced increase around -1.6 V. It is suggested to arise from $H_2$ evolution due to limitations of H incorporation (mass-transport), when $H_x NbO_{2.5}$ is formed on the surface. This also has been attributed to the increase in conductivity of the Nb oxide due to H intercalation. The H acts as a donor impurity, which increases the electronic conductivity of the Nb oxide.

After 200 cycles, the impedance spectrum completely changed character due to H intercalation into the Nb oxide. As H is incorporated, the oxide becomes more conductive, leading to decreased resistance and increased capacitance of the oxide layers.

Pacemaker characteristics

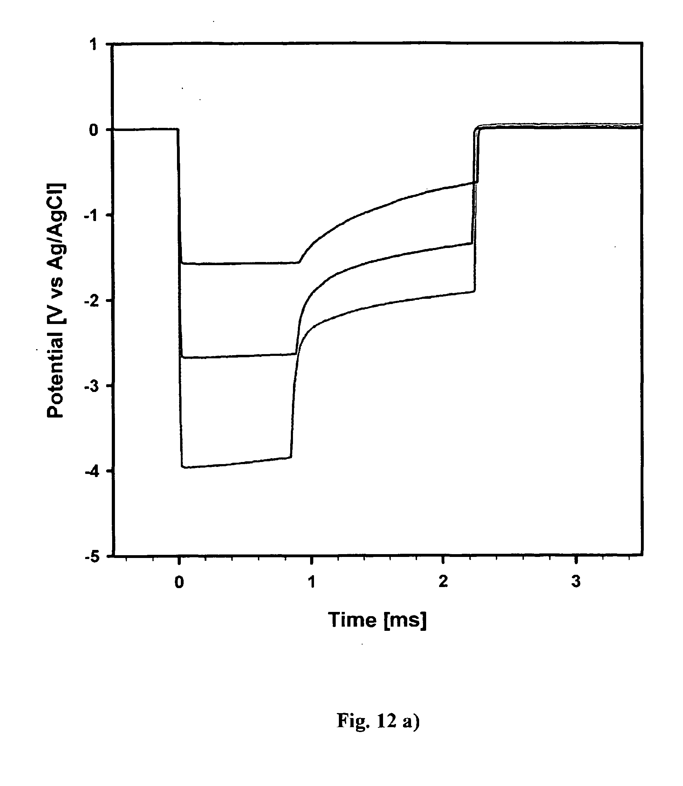

The out-put voltage of the pulses were set to -5, -7.5 and -10 V. Due to the potential drop caused by the electrolyte and the pores, the actual over-potential at the electrode was approximately -0.8, -1.6 and -2.7 V, respectively (relative to the open-circuit potential). The potential and current responses together with the recharging pulse following applied pulses of various magnitude are shown in Figs. 12a-b. The general shape of the curves is different from those reported previously for smooth and nano-porous carbon.

*Pacing polarization* - The electrode over-potential decreases only slightly with time during the pacemaker pulse even though the pulse is capacitively delivered. Fig. 9a, shows that the electrode over-potential decays insignificantly over the pulse duration for -0.80 and -1.6 V pulses while for the -2.6 and -4.0 V pulse potentials, the decay is -40 and -100 mV, respectively. Thus, little or no charge is transferred over the interface by faradaic reactions for the lower pulse potentials.

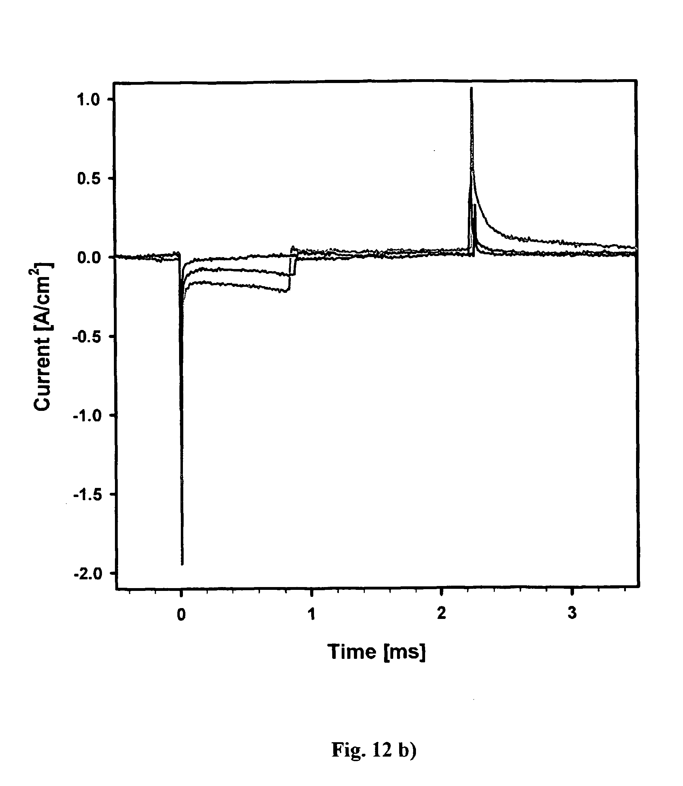

*Pacing current and impedance* - The current response is shown in Figure 12b. For -0.8 and -1.7 V potential pulses, the current is delivered in a peak at the first 0.1 ms of the pulse. The exponential decay of the current peak suggests that it originate from pure capacitive charge/discharging of the electrochemical double layer. For the higher potential pulses the peak is followed by a small (100 and 200 mA), almost constant current, that proceeds for as long as the out-put potential is applied. The magnitude of the small current increases with pulse time, implying a decrease of the oxide impedance as the pulse proceeds.

The pacing impedance of the electrode during the pulse can be calculated from the measured potential and current according to:

$$Z(t) = \frac{U(t)}{I(t)} \qquad\qquad (4)$$

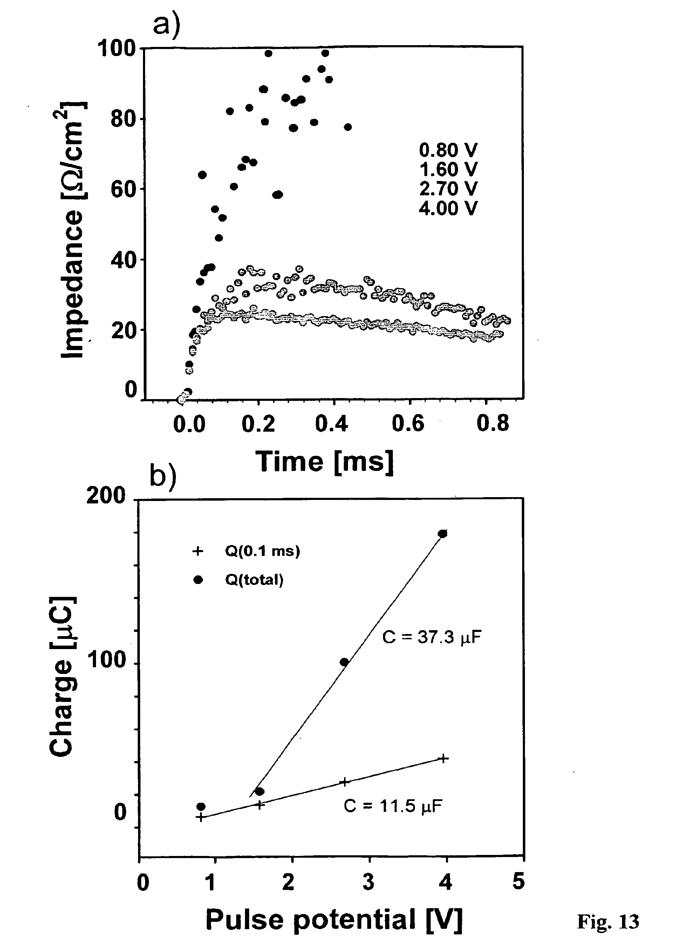

In Fig. 13a the pacing impedance is plotted vs. time during the pulse for different pulse potentials. For the first 0.1 ms, the impedance increases slightly for all pulse potentials. As the pulse proceeds the impedance of the -0.8 and -1.6 V pulses increase drastically while the impedance of the higher potential pulses reach a maximum and then decreases. The high impedance obtained for low pulse potentials can be explained by the lack of faradaic reactions. When the short current peak attributed to charging/discharging of the electrochemical double layer diminish, there is no current flowing across the interface, leading to an extremely high impedance according to equation 4. For higher pulse potentials, there are faradaic currents flowing throughout the entire pulse duration, as seen in Figure 12b.

If the cathodic current peaks at first 0.1 ms of the pulse are interpreted as originating from the charging/recharging of the electrochemical double layer, the capacitance of the electrode during pulsing can be obtained. In Figure 13b the charge delivered by the pulse versus the pulse potentials is plotted for both the stimulation pulse and the recharging pulse. The capacitance is obtained from the slope of the plot, and was 12 $\mu$lF/cm$^2$.

*After polarization* - The trailing edge voltage is the resulting iR-drop following the interruption of current passing the interface, which is determined by the resistance of the electrolyte and the current density. After the applied potential is released, the electrode interface commences to return to equilibrium with the electrolyte, i.e., relaxation process. The after-polarization (relaxation process) is dependent on the over-potential at the trailing edge and interfacial characteristics of the electrode.

*Recharging pulse* - The application of the recharging pulse does not polarise the electrode to an anodic potential, but merely brings it back to the original potential. The current response of the recharging pulse is clearly visible in Figure 12b. The origin of anodic current is the oxidation of hydrogen incorporated into the oxide.

Conclusions

The electrochemical properties of porous Nb$_2$O$_5$ electrodes, produced by plasma electrolytic oxidation, have been investigated in phosphate buffered saline solution. The oxide consists of two layers, one inner thin compact oxide (1.6-1.9 nm) and one thicker porous outer oxide. The interfacial electrochemical behaviour of the porous Nb$_2$O$_5$ is dependent on the magnitude and polarization of DC-bias applied to the electrode. At anodic polarization the capacitance of the inner barrier oxide layer decreases due to the increasing thickness of the layer. This leads to increased impedance of the oxide which shows passive behaviour during anodic polarization. At cathodic polarization the oxide changes properties due to intercalation of hydrogen, becomes more conductive and allows cathodic currents to flow. The charging/discharging mechanism remains mainly of capacitive character when the charge/discharge rate in increased. Porous Nb$_2$O$_5$ electrodes have extremely high interfacial impedance, and hence low energy loss, when transferring pacemaker pulses.

**Claims**

1.   Implantable electrode having an electrically conductive core comprising niobium covered by a stable biocompatible niobium oxide barrier layer, **characterized in that** said niobium oxide barrier layer comprises an inner compact niobium oxide and an outer porous niobium oxide.

**2.** Implantable electrode according to claim 1 where the porous niobium oxide has a pore size of 1 to 20 μm, preferably 2 to 15 μm, especially 3 to 10 μm.

**3.** Implantable electrode according to any of claims 1 to 2 where the thickness of the niobium oxide barrier layer is 0.5 to 15 μm, preferably 1 to 10 μm, especially 2 to 7 μm.

**4.** Implantable electrode according to any of claims 1 to 3 where the niobium oxide is niobium pentoxide.

**5.** Implantable electrode according to any of claims 1 to 4 where the core comprises a niobium layer.

**6.** Implantable electrode according to any of claims 1 to 4 where the core consists of niobium.

**7.** Implantable electrode according to any of claims 1 to 6 where the niobium oxide barrier layer has been produced by subjecting the core to high potential anodic pulses.

**8.** Implantable electrode according to any of claims 1 to 7 which is a pacemaker electrode or a defibrillator electrode.

**9.** A process of producing an implantable electrode comprising connecting an electrically conductive core of metal or metal alloy containing niobium as anode in an electrolyte, **characterized by** subjecting the core to high potential anodic pulses, having a pulse magnitude from 100 to 2000 V and a pulse duration from 1 to 20 ms to form a stable and biocompatible niobium oxide barrier layer on the core, where the niobium oxide barrier layer comprises an inner compact niobium oxide and an outer porous niobium oxide.

**10.** A process according to claim 9 where the core is made of essentially pure niobium.

**11.** A process according to claim 9 or 10 where the electrolyte is a phosphate buffered saline solution.

**12.** A process according to any of claims 9 to 11 where the electrolyte is a solution of calcium acetate and calcium glycerophosphate.

**13.** A process according to any of claims 9 to 12 where the pulse magnitude is 200 to 1000 V and especially 500 to 1000 V.

**14.** A process according to any of claims 9 to 13 where the pulse duration is 2 to 15 ms, especially about 7 to 13 ms.

**15.** A process according to any of claims 9 to 14 where the number of pulses is at least 40, preferably at least 50.

**16.** A process according to any of claims 9 to 15 where the number of pulses is at most 700, preferably at most 500, especially at most 300.

**17.** A process according to any of claims 9 to 16 where the number of pulses is 100 to 200.


**Patentansprüche**

**1.** Implantierbare Elektrode, die einen elektrisch leitfähigen Kern aufweist, der Niob umfasst und von einer stabilen biokompatiblen Nioboxid-Sperrschicht bedeckt ist, **dadurch gekennzeichnet, dass** die Nioboxid-Sperrschicht ein inneres kompaktes Nioboxid und ein äußeres poröses Nioboxid umfasst.

**2.** Implantierbare Elektrode nach Anspruch 1, wobei das poröse Nioboxid eine Porengröße von 1 bis 20 μm, vorzugsweise 2 bis 15 μm, insbesondere 3 bis 10 μm, aufweist.

**3.** Implantierbare Elektrode nach einem der Ansprüche 1 bis 2, wobei die Dicke der Nioboxid-Sperrschicht 0,5 bis 15 μm, vorzugsweise 1 bis 10 μm, insbesondere 2 bis 7 μm, beträgt.

**4.** Implantierbare Elektrode nach einem der Ansprüche 1 bis 3, wobei das Nioboxid Niobpentoxid ist.

**5.** Implantierbare Elektrode nach einem der Ansprüche 1 bis 4, wobei der Kern eine Niobschicht umfasst.

**6.** Implantierbare Elektrode nach einem der Ansprüche 1 bis 4, wobei der Kern aus Niob besteht.

**7.** Implantierbare Elektrode nach einem der Ansprüche 1 bis 6, wobei die Nioboxid-Sperrschicht hergestellt wurde, indem der Kern anodischen Impulsen mit hoher Spannung ausgesetzt wurde.

**8.** Implantierbare Elektrode nach einem der Ansprüche 1 bis 7, die eine Schrittmacherelektrode oder eine Defibrillatorelektrode ist.

**9.** Verfahren zur Herstellung einer implantierbaren Elektrode, umfassend das Anschließen eines elektrisch leitfähigen Kerns aus Metall oder einer Metalllegierung, der Niob enthält, als Anode in einem Elektrolyten, **gekennzeichnet durch** das Aussetzen des Kerns gegenüber anodischen Impulsen mit hoher Spannung, die einen Impulsbetrag von 100 bis 2000 V und eine Impulsdauer von 1 bis 20 ms aufweisen, um eine stabile und biokompatible Nioboxid-Sperrschicht auf dem Kern zu bilden, wobei die Nioboxid-Sperrschicht ein inneres kompaktes Nioboxid und ein äußeres poröses Nioboxid umfasst.

**10.** Verfahren nach Anspruch 9, bei dem der Kern aus im Wesentlichen reinem Niob hergestellt wird.

**11.** Verfahren nach Anspruch 9 oder 10, bei dem der Elektrolyt eine phosphatgepufferte Kochsalzlösung ist.

**12.** Verfahren nach einem der Ansprüche 9 bis 11, bei dem der Elektrolyt eine Lösung aus Calciumacetat und Calciumglycerophosphat ist.

**13.** Verfahren nach einem der Ansprüche 9 bis 12, bei dem der Impulsbetrag 200 bis 1000 V und insbesondere 500 bis 1000 V, beträgt.

**14.** Verfahren nach einem der Ansprüche 9 bis 13, bei dem die Impulsdauer 2 bis 15 ms, insbesondere etwa 7 bis 13 ms, beträgt.

**15.** Verfahren nach einem der Ansprüche 9 bis 14, bei dem die Anzahl der Impulse mindestens 40, vorzugsweise mindestens 50, beträgt.

**16.** Verfahren nach einem der Ansprüche 9 bis 15, bei dem die Anzahl der Impulse höchstens 700, vorzugsweise höchstens 500, insbesondere höchstens 300, beträgt.

**17.** Verfahren nach einem der Ansprüche 9 bis 16, bei dem die Anzahl der Impulse 100 bis 200 beträgt.


**Revendications**

**1.** Électrode implantable présentant un noyau électriquement conducteur comprenant du niobium recouvert d'une couche barrière d'oxyde de niobium biocompatible stable, **caractérisée en ce que** ladite couche barrière d'oxyde de niobium comprend un oxyde de niobium interne compact et un oxyde de niobium externe poreux.

**2.** Électrode implantable selon la revendication 1, dans laquelle l'oxyde de niobium poreux présente une taille de pore de 1 à 20 $\mu$m, de préférence de 2 à 15 $\mu$m, en particulier de 3 à 10 $\mu$m.

**3.** Électrode implantable selon l'une quelconque des revendications 1 à 2, dans laquelle l'épaisseur de la couche barrière d'oxyde de niobium est de 0,5 à 15 $\mu$m, de préférence de 1 à 10 $\mu$m, en particulier de 2 à 7 $\mu$m.

**4.** Électrode implantable selon l'une quelconque des revendications 1 à 3, dans laquelle l'oxyde de niobium est du pentoxyde de niobium.

**5.** Électrode implantable selon l'une quelconque des revendications 1 à 4, dans laquelle le noyau comprend une couche de niobium.

**6.** Électrode implantable selon l'une quelconque des revendications 1 à 4, dans laquelle le noyau se compose de niobium.

**7.** Électrode implantable selon l'une quelconque des revendications 1 à 6, dans laquelle la couche barrière d'oxyde de niobium a été produite en soumettant le noyau à des impulsions anodiques haute tension.

**8.** Électrode implantable selon l'une quelconque des revendications 1 à 7, qui est une électrode de stimulateur cardiaque ou une électrode de défibrillateur.

**9.** Procédé de fabrication d'une électrode implantable comprenant la connexion d'un noyau électriquement conducteur en métal ou alliage métallique contenant du niobium en tant qu'anode dans un électrolyte, **caractérisé par** la soumission du noyau à des impulsions anodiques haute tension, présentant une hauteur d'impulsion allant de 100 à 2000 V et une durée d'impulsion allant de 1 à 20 ms pour former une couche barrière d'oxyde de niobium biocompatible et stable sur le noyau, la couche barrière d'oxyde de niobium comprenant un oxyde de niobium interne compact et un oxyde de niobium externe poreux.

**10.** Procédé selon la revendication 9, dans lequel le noyau est fabriqué en niobium essentiellement pur.

**11.** Procédé selon la revendication 9 ou 10, dans lequel l'électrolyte est une solution saline tamponnée au phosphate.

**12.** Procédé selon l'une quelconque des revendications 9 à 11, dans lequel l'électrolyte est une solution d'acétate de calcium et glycérophosphate de calcium.

**13.** Procédé selon l'une quelconque des revendications 9 à 12, dans lequel la hauteur d'impulsion est de 200 à 1000 V et en particulier de 500 à 1000 V.

**14.** Procédé selon l'une quelconque des revendications 9 à 13, dans lequel la durée d'impulsion est de 2 à 15 ms, en particulier d'environ 7 à 13 ms.

**15.** Procédé selon l'une quelconque des revendications 9 à 14, dans lequel le nombre d'impulsions est au moins de 40, de préférence au moins de 50.

**16.** Procédé selon l'une quelconque des revendications 9 à 15, dans lequel le nombre d'impulsions est au plus de 700, de préférence au plus de 500, en particulier au plus de 300.

**17.** Procédé selon l'une quelconque des revendications 9 à 16, dans lequel le nombre d'impulsions est de 100 à 200.

5 μm

**Fig. 1**

Fig. 2

a)

b)

20 μm

20 μm

c)

d)

20 μm

20 μm

Fig. 3

**Fig. 4**

Fig. 5

Fig. 6

**Fig. 7**

Fig. 8

Fig. 9

**Fig. 10**

a)

b)

Fig. 11

Fig. 12 a)

**Fig. 12 b)**

a)

b)

Fig. 13

Fig. 14

Fig. 15

a)

10 μm

b)

10 μm

c)

10 μm

d)

10 μm

e)

10 μm

f)

10 μm

Fig. 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **H. Zilleen ; E. Ivers-Tiffee.** Niobium as new material for electrolyte capacitors with nanoscale dielectric oxide layers. *Proceedings of the IEEE International Conference on Properties and Applications of Dielectric Materials,* 2003, vol. 3, 1134-1137
- **A. L. Yerokhin ; X. Nie ; A. Leyland ; A. Matthews ; S. J. Dowey.** Plasma electrolysis for surface engineering. *Surface and Coatings Technology,* 15 December 1999, vol. 122 (2-3), 73-93
- **A. Norlin ; J. Pan ; C. Leygraf.** Investigation of Electrochemical Behavior of Stimulation/Sensing electrode materials - I. Pt, Ti, and TiN-Coated Electrodes. *Journal of Electrochemical Society,* 2004
- **A. Norlin ; J. Pan ; C. Leygraf.** Investigation of Electrochemical Behavior of Stimulation/Sensing electrode materials - II. Conductive Oxide Coated Electrodes. *Journal of Electrochemical Society,* 2004